# EUROPEAN PATENT SPECIFICATION

(11) **EP 2 811 893 B1**
(45) Date of publication and mention of the grant of the patent: **01.02.2017**
(21) Application number: 13705681.8
(22) Date of filing: 07.02.2013
(51) Int. Cl.: A61B 5/00, A61C 17/00

(54) **ORAL HEALTH DETECTION DEVICE**
VORRICHTUNG ZUM MUNDGESUNDHEITSNACHWEIS
DISPOSITIF DE DÉTECTION DE SANTÉ ORALE

(30) Priority: 07.02.2012 US 201261595809 P
(43) Date of publication of application: 17.12.2014
(73) Proprietor: Braun GmbH, 61476 Kronberg/Taunus (DE)
(72) Inventor: KRESSMAN, Frank Peter, 61476 Kronberg (DE); ALMEDOM, Ruta, 61476 Kronberg (DE); MAO, Xiaole, Cincinnati Ohio 45201 (US)
(74) Representative: Schneider, Stefan Michael
(86) International application number: PCT/US2013/025097
(87) International publication number: WO 2013/119776

(56) References cited:
- WO-A1-2009/130464
- WO-A1-2010/023582
- US-A- 5 894 620
- US-A- 6 024 562
- US-A1- 2004 019 990
- US-A1- 2011 318 712

## Description

### FIELD OF THE INVENTION

The present disclosure relates generally to an oral health detection device, and more particularly to an oral health detection device that uses an excitation radiation for the detection of dental deposits on teeth, such as plaque, caries, bacterial infections and tartar, during normal brushing regimen.

### BACKGROUND OF THE INVENTION

Current practice is that consumers retrieve desired information about their oral hygiene during dental visits. Consumers lack tool which provide the ability to independently assess and control their brushing habits and receive information concerning their oral health while at home. Self-diagnosis at home has significant limitations, including low visual accessibility of the teeth and mouth and inability to understand signals of most oral care issues early on before they become severe. Areas of teeth having dental deposits are known to respond to light by issuing fluorescent radiation that differs in intensity and spectral distribution from radiation returned from a healthy tooth. Reflected radiation may, therefore, be used to evaluate oral health.

Document US 5 894 620 discloses an oral health device in accordance with the preamble of claim 1, where the excitation radiation has a wavelength between 400 nm and 500 nm and the light sensor is arranged to receive fluorescence radiation having a wavelength between 500 nm and 600 nm.

Accordingly, there is a need for an oral health detection device that rapidly and effectively communicates oral health assessment and provides feedback to guide consumers during their normal brushing regimen.

### SUMMARY OF THE INVENTION

In the embodiment of the invention, an oral health detection device for investigation of dental deposits is provided. The device includes a cleaning section having a cleaning head portion; a handle section having a motor and a drive shaft having a longitudinal axis; a light source for emitting excitation radiation having a wavelength greater than about 580 nm; an assembly for directing the excitation radiation to the dental region of the mouth; a light sensor for receiving fluorescence radiation having a wavelength greater than about 850 nm reflected from dental deposits; a logic component operable to analyze the fluorescence radiation received by the light sensor; and a display for providing to a user information concerning the presence of dental deposits. The cleaning head portion includes at least one light probe which is static relative to the handle section and a plurality of cleaning elements arranged generally transverse to the longitudinal axis such that a cleaning motion of the plurality of cleaning elements includes a back and forth oscillating movement of the plurality of cleaning elements about the longitudinal axis and relative to the light probe.

In another embodiment not part of the invention, a handle section for an oral health detection device that is capable of use with both diagnostic and non-diagnostic cleaning sections is provided. The handle section includes a motor and a drive shaft having a longitudinal axis; a coupling section at an end of the handle section for coupling a cleaning section thereto; a light source for emitting excitation radiation having a wavelength greater than about 580 nm; an assembly for directing the excitation radiation to the dental region of the mouth; a light sensor for receiving fluorescence radiation having a wavelength greater than about 850 nm reflected from dental deposits; a logic component operable to analyze the fluorescence radiation received by the light sensor; and a controller including a light source activating device that is activated when a diagnostic cleaning section including a coding device is coupled to the coupling section.

In yet another embodiment not part of the invention, a cleaning section for use with a handle section is provided. The cleaning section includes a cleaning head portion having at least one light probe; a coupling section to effect coupling of the cleaning section to a handle section; and a coding device for providing a signal that enables activation of a light source contained within a handle section.

### BRIEF DESCRIPTION OF THE DRAWINGS

While the specification concludes with claims which particularly point out and distinctly claim the subject matter that is regarded as the invention, it is believed the various embodiments will be better understood from the following description taken in conjunction with the accompanying drawings, in which:
Fig. 1 depicts an electric oral health detection device according to one or more embodiments shown and described herein;
Fig. 2 depicts a schematic, cross sectional representation of an electric oral health detection device according to one or more embodiments shown and described herein;
Fig. 3 depicts the interface between a handle and a cleaning section of an electric oral health detection device according to one or more embodiments shown and described herein;
Fig. 4 depicts a schematic, cross sectional representation of an electric oral health detection device according to one or more embodiments shown and described herein;
Fig. 5 depicts an oral health detection system according to one or more embodiments shown and described herein;
Fig. 6 depicts a schematic, cross sectional representation of an electric oral health detection device having an indication element according to one or more embodiments shown and described herein;
Figs. 7A-7C schematically depict various configurations of a cleaning head section of an electric oral health detection device according to one or more embodiments shown and described herein;
Figs. 8A-8B depict schematic, cross sectional representations of various configurations of a cleaning head section of an electric oral health detection device according to one or more embodiments shown and described herein;
Fig. 9 depicts a schematic, cross sectional representation of a cleaning head section according to one or more embodiments shown and described herein;
Fig. 10 depicts a schematic, cross sectional representation of a cleaning head section according to one or more embodiments shown and described herein;
Figs. 11A-11B graphically depict the intensity of the fluorescence radiation for a dental deposit and tooth enamel according to one or more embodiments shown and described herein;
Fig. 11C graphically depicts the intensity of the fluorescence radiation for exemplary dyes used in oral care compositions according to one or more embodiments shown and described herein;
Fig. 12 graphically depicts the intensity of the fluorescence noise for oral care compositions with and without color dyes according to one or more embodiments shown and described herein;
Fig. 13 graphically depicts the reduction in the intensity of the fluorescence noise for oral care compositions with color dyes according one or more embodiments shown and described herein;
Fig. 14A graphically depicts the intensity of the fluorescence radiation for an exemplary red dye used in oral care compositions according to one or more embodiments shown and described herein;
Fig. 14B graphically depicts the intensity of the fluorescence radiation for an exemplary yellow dye used in oral care compositions according to one or more embodiments shown and described herein; and
Fig. 14C graphically depicts the intensity of the fluorescence radiation for an exemplary blue dye used in oral care compositions according to one or more embodiments shown and described herein.

### DETAILED DESCRIPTION OF THE INVENTION

The following text sets forth a broad description of numerous different embodiments of the present disclosure. The description is to be construed as exemplary only and does not describe every possible embodiment since describing every possible embodiment would be impractical, if not impossible. It will be understood that any feature, characteristic, component, composition, ingredient, product, step or methodology described herein can be deleted, combined with or substituted for, in whole or part, any other feature, characteristic, component, composition, ingredient, product, step or methodology described herein. Numerous alternative embodiments could be implemented, using either current technology or technology developed after the filing date of this patent, which would still fall within the scope of the claims. All publications and patents cited herein are incorporated herein by reference.

Although the embodiments are described herein in the context of an electric oral heath detection device, such as an electric toothbrush, embodiments are not limited thereto. Embodiments disclosed herein may be implemented in a wide-variety of applications, such as in the application of a manual toothbrush, powered flossers, gum massagers and many other devices with or without cleaning elements.

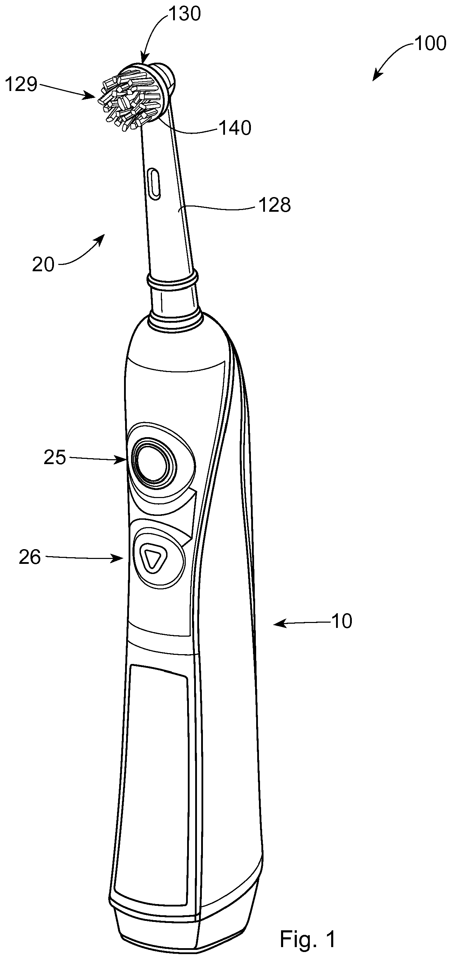

Referring now to FIG. 1, an exterior view of one embodiment of an electric oral health detection device is illustrated. In general terms, the electric oral health detection device is an optical investigation device that irradiates a tooth tissue region with an excitation radiation, in response to which a response radiation is issued from the tooth tissue region and evaluated to determine the presence of dental deposits, including, for example, plaque, caries, bacterial infections and tartar.

In one embodiment, the electric oral health detection device may be configured as an electric toothbrush 100 having a cleaning section 20 and a elongated body or handle section 10 such that toothbrush 100 may be used by a consumer for maintaining oral hygiene by brushing and, in addition, for monitoring oral health as described in further detail below.

The cleaning section 20 is discussed in more detail below. In one embodiment, the handle section 10 may include a first switch 25 operable to control the ON/OFF condition of the toothbrush 100 and a second switch 26 operable to select one of a plurality of possible modes of operation programmed in the toothbrush 100. In one embodiment, second switch 26 may be used to switch between a plurality of different modes of operation of toothbrush 100, for example, a regular cleaning mode, a massage or deep cleaning mode, a soft or sensitive mode for sensitive areas, a massage mode for gentle stimulation of gums and a polish mode for whitening. In another embodiment, second switch 26 or an additional switch may be operable to select between a brushing mode and an examination mode. In one embodiment, first switch 25 and second switch 26 are pushbutton switches; different switches, however, can be used including for example, slidable switches or any other type of suitable switch. Additionally, the toothbrush 100 may have an "auto-on" switch which when the user presses the cleaning section 20 against their teeth, the toothbrush 100 turns on. When the user pulls the toothbrush 100 away from their teeth and the pressure is released, the toothbrush 100 turns off.

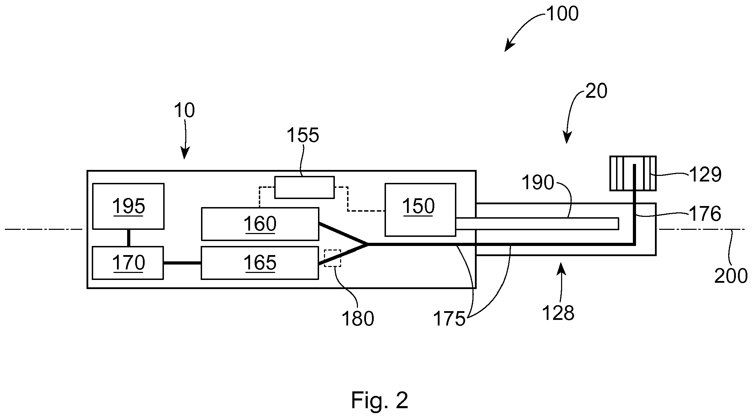

Referring now to FIG. 2, a graphic illustration of one embodiment of an electric oral health detection device implemented as an electric toothbrush. It should be understood that the arrangement of the components of the electric toothbrush 100 is for illustrative purposes only and embodiments are not limited to such arrangement of components or configurations of the illustrated electric toothbrush 100. The electric toothbrush 100 includes a handle section 10 and a cleaning section 20, for example, a refill. The cleaning section 20 may be coupled to the handle section 10 at a coupling section. In one embodiment, the cleaning section 20 is removably coupled to the handle section 10 such that cleaning sections of differing configurations may be attached to the handle section 10. For example, in one embodiment, a cleaning section may include optical components for the detection of dental deposits. In another embodiment, a cleaning section including various other designs may be included, for example, a child's brush design with soft cleaning elements, an adult's brush design with hard cleaning elements, an interproximal brush design or the like.

In another embodiment, the cleaning section 20 is not removable from the handle section 10 such that the handle section 10 and the cleaning section 20 are one integral component, such as a manual toothbrush, for example. The handle section 10 may be made of non-electrically conductive material, such as molded plastic, for example.

The illustrated cleaning section 20 generally includes an elongated housing or neck portion 128 extending along a longitudinal axis 200 and a cleaning head portion 129 for insertion into the oral cavity. The elongated housing 128 may include a profile ring having an inside contour complementary with an outside contour of the handle section 10. In this manner, the cleaning section 20 can be push-fitted onto the handle section 10 in a manner preventing relative rotation of the cleaning section 20 with respect to the handle section 10. A tab/slot, key/spline or other similar structure may be included in the corresponding contour surfaces to facilitate alignment of the cleaning section 20 with the handle section 10 and to further prevent relative rotation between the two. The cleaning head portion 129 is mounted such that it can in operation be driven into a rotation or oscillating rotation around a rotation axis when the cleaning section 20 is attached to the handle section 10. Many different kinds of cleaning motions, including rotary, oscillating, vertical and/or horizontal sweeping and the like, may be used. Generally, as used herein, cleaning motion describes any desired or effective movement of the cleaning elements or bristles relative to other components in the toothbrush 100 to affect cleaning.

As shown in Fig. 1, the cleaning head portion 129 has a substantially circular shape, although it may alternatively have a generally elliptical, rectangular, oblong, oval or other suitable shape. In some embodiments, the cleaning head portion 129 includes a carrier 130 which supports a plurality of cleaning elements 140 that are mounted to the carrier 130. The cleaning head portion 129 is mounted such that it can in operation be driven into a rotation or oscillating rotation around a rotation axis when the cleaning section 20 is attached to a handle section 10. Any suitable method of mounting the cleaning elements 140 to the carrier 130 may be used. For example, where the cleaning elements 140 comprise a plurality of bristles, methods such as hot tufting, gluing, stapling, and the like, may be utilized. As another example, where the cleaning elements 140 comprise a plurality of elastomeric elements, methods such as gluing, snap-fitting, welding, molding, etc. may be utilized.

The term "cleaning elements" is used to refer to any suitable element which can be inserted into the oral cavity. Some suitable elements include bristle tufts, elastomeric massage elements, elastomeric cleaning elements, massage elements, tongue cleaners, soft tissue cleaners, hard surface cleaners, combinations thereof, and the like. The cleaning elements 140 may include a wide variety of materials and may have a number of different configurations. Any suitable material and/or any suitable configuration may be utilized. For example, in some embodiments, the cleaning elements1 40 may comprise tufts. The tufts may comprise a plurality of individual filaments which are securely attached to a cleaning element carrier. Such filaments may be polymeric and may include polyamide or polyester or a thermoplastic elastomeric polyamide grind or mixtures thereof. The longitudinal and cross sectional dimensions of the filaments and the profile of the filament ends can vary. Additionally, the stiffness, resiliency and shape of the filament end can vary. Some examples of suitable dimensions include a length between about 6.0 mm and about 10 mm and in another embodiment between about 7.0 mm and about 8.5 mm, or any individual number within these ranges. Additionally, the filaments may include a substantially uniform cross-sectional dimension of between about 100 to about 350 microns, in another embodiment in a range of between about 125 microns and about 175 microns, or any individual number within these ranges. The tips of the filaments may be any suitable shape, examples of which include a smooth tip, a rounded tip, tapered and a pointed tip. In some embodiments, the filaments may include a dye which indicates wear of the filaments as described in U.S. Patent No. 4,802,255. Other suitable examples of filaments are described in U.S. Patent No. 6,018,840. In some embodiments, the cleaning element fields may comprise fins as described in U.S. Patent No. 6,553,604, and U.S. Patent Application Publication Nos. 2004/0177462; 2005/0235439; and 2005/0060822, which are hereby incorporated by reference in their entirety. In some embodiments, the cleaning element fields may comprise a combination of fins and tufts.

In one embodiment, the head may comprise a variety of cleaning elements. For example, the cleaning head portion 129 may comprise bristles, abrasive elastomeric elements, elastomeric elements in a particular orientation or arrangement, for example, pivoting fins, prophy cups, or the like. Some suitable examples of elastomeric cleaning elements and/or massaging elements are described in U.S. Patent Application Publication Nos. 2007/0251040; 2004/0154112; 2006/0272112; and in U.S. Patent Nos. 6,553,604; 6,151,745. The cleaning elements may be tapered, notched, crimped, dimpled, or the like. Some suitable examples of these cleaning elements and/or massaging elements are described in U.S. Patent Nos. 6,151,745; 6,058,541; 5,268,005; 5,313,909; 4,802,255; 6,018,840; 5,836,769; 5,722,106; 6,475,553; and U.S. Patent Application Publication No. 2006/0080794.

The cleaning head portion 129 may comprise a soft tissue cleanser constructed of any suitable material. The soft tissue cleanser may comprise any suitable soft tissue cleansing elements. Some examples of such elements as well as configurations of soft tissues cleansers on a toothbrush are described in U.S. Patent Application Nos. 2006/0010628; 2005/0166344; 2005/0210612; 2006/0195995; 2008/0189888; 2006/0052806; 2004/0255416; 2005/0000049; 2005/0038461; 2004/0134007; 2006/0026784; 20070049956; 2008/0244849; 2005/0000043; 2007/140959; and U.S. Patent Nos. 5,980,542; 6,402,768; and 6,102,923.

Maintained within handle section 10 are various components that produce the electro-optical means of the toothbrush 100, including an actuator 150, a power source 155, light source 160, a sensor 165, a logic component 170, a light guide 175, a light probe 176, filter 180 and an amplifier (not shown).

The actuator 150 is operatively connected to the cleaning head portion 129. The actuator 150 may produce a linear, rotational, gyrating, orbital or vibratory motion which is transferred to the cleaning head portion 129 via a drive mechanism or shaft 190. The actuator 150 may include an electric motor, a piezoelectric motor, electro-chemical polymer driven motor, any other suitable motor, or any combination thereof. The actuator 150 may be capable of converting electrical energy (for example, from the power source 155) into motion energy in order to operate the cleaning head portion 129 as described herein. For example, in one embodiment, the actuator 150 may be a rotary electrical motor which is capable of producing rotational motion. The actuator 150 may be coupled to the cleaning head portion 129 via the drive mechanism 190 having one or more gears, axles, belts, drive shafts, other suitable components, or any combination thereof.

In one embodiment, the drive mechanism or shaft 190 is operatively connected to the cleaning head portion 129 when some action by the actuator 150 results in a response in the cleaning head portion 129. The shaft 190 may protrude from the end of the handle section 10 and adapted to be received by a complementary coupling portion of the cleaning section 20. The shaft 195 may rotate, oscillate, linearly reciprocate, gyrate, vibrate or orbit when driven by the actuator 150 in order to impart one or more motions to the cleaning head portion 129 and to the plurality of cleaning elements 140.

In another embodiment, the plurality of cleaning elements 140 can oscillate back and forth angularly to provide a cleaning action substantially similar to an up-down manual brushing action. In one embodiment, the plurality of cleaning elements 140 can oscillate at a frequency from about 75 Hz to about 300 Hz, or any individual number within the range. In one embodiment, the cleaning head portion 129 can operate in the sonic frequency range, for example 262 ± 30 Hz. The amount of angular movement as well as the speed exhibited by the cleaning head portion 129 and the plurality of cleaning elements 140 can impact the efficacy of the cleaning action. Generally, oscillation angle within the range of about 40-60 degrees is considered beneficial. For example, the cleaning head portion 129 may move through an angle of about 44 degrees, i.e., +/- 22 degrees relative to the carrier 130, in some embodiments. Another example includes about 55 degrees angle. However, any suitable angle may be utilized. For example, other angles greater than about 55 degrees or less than about 44 degrees may be used.

In some embodiments, the cleaning head portion 129 can move through an angle of from about 10 degrees to about 90 degrees, or any individual number within the range. In some embodiments, the cleaning head portion 129 can move through an angle greater than about 10 degrees, greater than about 12 degrees, greater than about 15 degrees, greater than about 18 degrees, greater than about 20 degrees, greater than about 22.5 degrees, greater than about 25 degrees, greater than about 30 degrees, greater than about 35 degrees, greater than about 40 degrees, greater than about 45 degrees, greater than about 50 degrees, greater than about 55, greater than about 60 degrees, greater than about 65 degrees, greater than about 70 degrees, greater than about 75 degrees, greater than about 80 degrees, greater than about 85 degrees, and/or less than about 90 degrees, less than about 85 degrees, less than about 80 degrees, less than about 75 degrees, less than about 70 degrees, less than about 65 degrees, less than about 60 degrees, less than about 55 degrees, less than about 50 degrees, less than about 45 degrees, less than about 40 degrees, less than about 35 degrees, less than about 30 degrees, less than about 25 degrees, less than about 22.45 degrees, less than about 20 degrees, less than about 18 degrees, less than about 15 degrees, less than about 12 degrees, or less than about 10 degrees.

A gearing arrangement can be provided between the actuator and the drive mechanism or between the drive mechanism and the cleaning head portion 129 in order to impart motion thereto. A suitable motor and mechanical linkage transmission system is disclosed for example in U.S. Patent Application Publication No. 2008/0307591 to Farrell et al., and USPNs 6,360,395 and 5,617,601.

Power source 155 may permit the toothbrush 100 to operate wirelessly, that is, without having a wire or a cable leading to another source of power such as, for example, a common household 110-Volt electrical outlet. The power source 155 may be, for example, a rechargeable or non-rechargeable battery. A rechargeable battery may employ lithium-ion or nickel-metal hydride technology, and a non-rechargeable battery may employ alkaline or zinc-carbon technology. Other types of rechargeable and non-rechargeable battery technologies may be used as well, including those presently known and those yet to be developed. In addition to batteries, the power source 155 may comprise other types of energy sources as well. Alternatively, the electric toothbrush 100 may be connected to an external power source for powering the actuator 150.

A user exposes dental deposits to emitted electromagnetic energy from light source 160. Without wishing to be bound by theory, it is believed that the dental deposits absorb at least a portion of the electromagnetic energy and reflect a portion of that electromagnetic energy. The dental deposit also emits electromagnetic radiation having a different wavelength or range of wavelengths than that of the electromagnetic energy emitted by light source 160. The autofluorescence may produce detectable wavelength contrast between clean tooth surfaces and the dental deposits.

Referring to Fig. 2, as light source 160 emits excitation radiation toward and/or into the oral cavity, a portion of that energy may reflect (reflected energy) from oral cavity surfaces such as teeth, gums and a tongue. In addition, as set forth above, a portion of the energy transmitted from light source 160 may be absorbed by dental deposits within the oral cavity at a location having a particular condition (for example, at a plaque location). At least a portion of the absorbed energy may be emitted by the dental deposit as fluorescent energy, thereby highlighting a condition within the oral cavity (for example, plaque buildup).

In one embodiment, the light source 160 for the generation of the excitation radiation may be any suitable electromagnetic energy source. Some non-limiting examples include a light-emitting element. A wide variety of light-emitting elements may be used with the present invention. For example, the light-emitting elements can be a small, low power consumption, light emitting diodes (LEDs) such as those commercially available under the designation Luxeon™ manufactured by Lumileds Lighting, LLC of San Jose CA. Other commercially available light-emitting elements include those from American Opto Plus LED Corp. and from LiteOn Corp. sold under the tradename LTL42TBKL14-1B2. The LED can operate from a relatively low voltage DC power supply, such as greater than about 0.1 volts to about 9 volts. In some embodiments, the LED may operate from a voltage of greater than about 0.1 volts, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.5, 3, 3.5, 4, 4.5, 5, 5.5, 6.0, 6.5, 7, 7.5, 8, 8.5, and/or less than about 9, 8.5, 8, 7.5, 7, 6.5, 6, 5.5, 5, 4.5, 4, 3.5, 3, 2.5, 2, 1.9, 1.8, 1.7, 1.6, 1.5, 1.4, 1.3, 1.2, 1.1, 1, .9, .8, .7, .6, .5, .4, .3, .2, or 0.1 volts. The light-emitting element may have a diameter of greater than about 0.5, 1, 2, 3, 4, 5, 6, 7, 8, 10, 15, 20 mm and/or less than about 20, 15, 10, 8, 7, 6, 5, 4, 3, 2, or 1 mm. Additional examples of light emitting elements include, but are not limited to, laser diodes, gas lasers, dye lasers, solid state lasers, semi-conductor lasers and flash lamps.

Additionally, suitable electromagnetic energy sources may emit a wide variety of energy intensities. Any suitable intensity may be utilized. There are several parameters which may be utilized to identify the intensity, flux density, etc., of the energy emission from the LED. For example, Flux Density at a Representative Tooth Surface (FDRT), Percent Total Luminous Flux Within a Solid Angle, Half Angle and/or Viewing Angle, Emission Temperature, and Power Dissipation, can be measured in accordance with the procedure described in U.S. Patent Application Publication No. 2005/0053895.

The function of the toothbrush 100 in accordance with the present disclosure will be further explained herein using the example of fluorescence diagnosis, in which the fluorescence radiation arising at the tooth surface in reaction to the irradiation is evaluated. It is also possible to use other wavelengths for the excitation and/or response radiation.

In one embodiment, the light source emits excitation radiation consisting of red light having a spectrum in the wavelength range of from about 580 to about 800 nm, or any individual number within the range. In another embodiment, the excitation radiation may lie in the range between about 580 nm and about 640 nm; in another example between about 640 nm and about 680 nm; in another example between about 680 nm and about 740 nm; and in yet another example between about 740 nm and about 800 nm. In another embodiment, the excitation wavelength may be about 633 nm or about 655 nm or about 780 nm. In one embodiment, the response wavelength range is from about 680 nm to about 1100 nm, or any individual number within the range. In another embodiment, the response radiation may lie in the range between about 680 nm and about 800 nm; in another example between about 800 nm and about 950 nm. In another embodiment, the response radiation may be about 740 nm or about 900 nm or about 910 nm.

The light source 160 may include a meter, such as a potentiometer, for adjusting the light intensity as needed.

In addition to the handle section, light source 160 may be disposed in any suitable location on toothbrush 100. For example, light source 160 may be disposed in the head or the neck of toothbrush 100. Additionally, embodiments are contemplated where toothbrush 100 comprises more than one light source 160. In such embodiments, a second light source may have an emission spectrum which is different than that of light source 160, thereby inducing fluorescence in a dental deposit which is not induced by light source 160.

In one embodiment, the excitation radiation is generated by the light source 160 and coupled into at least one light guide 175 configured for at least one of directing the excitation radiation from the light source 160 onto a tooth surface and receiving the return or response radiation from a tooth surface. In one embodiment, the at least one light guide may be an individual optical fiber or a plurality of individual optical fibers. In another embodiment, the light guide 175 may be fork or Y-shaped with a first part for guiding the excitation radiation and a second part for guiding the return radiation. In another embodiment, the light guide 175 may be fabricated as a light pipe from plastic or glass, or other suitable structure operable to guide excitation from the light source 160 onto a tooth surface and to receive or guide the return radiation from a tooth surface. The light guide 175 may also have any suitable length, width or other dimensions as needed or desired.

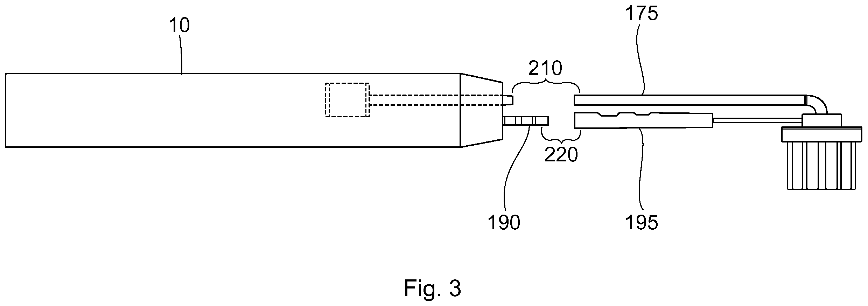

As shown in Fig. 3, in one embodiment, a portion of the light guide 175 is within the handle section 10 and a portion is within the neck portion 128 of the cleaning section 20. In one embodiment, the portion of the light guide 175 that is within the neck portion 128 is separate from and parallel to the drive shaft 190. As a result, there exists two interfaces between the handle section 10 and the cleaning section 20, an optical interface 210 and a mechanical interface 220. The optical interface 200 is the interface between the portion of the light guide 175 located in the handle section 10 and the portion of the light guide located in the cleaning section 20. The mechanical interface 210 is the interface between the drive shaft 190 and a gearing arrangement 195 located in the neck portion 128. In one embodiment, the optical interface 210 includes a mechanism to prevent interference of transmission of light caused by toothpaste or saliva building up on the interface 210, for example, coating the optical interface with a water repellant or hydrophobic agent.

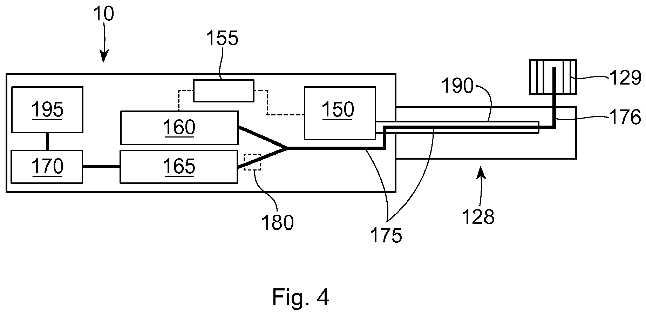

In another embodiment, such as shown in Fig. 4, the portion of the light guide 175 that is within the neck portion 128 is integrated into the drive shaft 195. The drive shaft 195 may have a portion that is hollowed out to allow the light guide 175 to be disposed within the drive shaft 195. In this embodiment, there exists one interface between the handle section 10 and the cleaning section 20, a combined optical and mechanical interface 230.

Connected to the light guide 175 is a light probe 176 that extends from the neck portion 128 into and through the cleaning head portion 129. In one embodiment, a portion of the light probe 176 is located within the neck portion 128 and a portion is within the cleaning head portion 129. Thus, during operation of the toothbrush 100, excitation radiation from the light source 160 is directed through the light guide 175 and into the light probe 176 in order to reach a tooth surface. Similarly, the return radiation is guided back through the light probe 176 and into the light guide 175.

As explained in more detail below, in one embodiment, the light probe 176 does not extend beyond the height of the plurality of cleaning elements 140 that are mounted to the carrier 130. In another embodiment, the light probe 176 may extend beyond the height of the plurality of cleaning elements 140 that are mounted to the carrier 130. In one embodiment, the light probe 176 may be an individual optical fiber, a plurality of individual optical fibers, a glass or plastic rod-like structure or a plurality of structures. In another embodiment, the light probe 176 can be a two component material such that the portion within the neck portion is made of a different material, for example plastic or glass, than the portion within the cleaning head portion 129, for example, a soft polymeric material. In another embodiment, the light probe 176 can be made of various polymer optical materials such as cyclic olefin copolymer, cycloolefin polymer, polycarbonate, poly(methyl methacrylate), polystyrene, allyl diglycol carbonate, poly(styrene-co-acrylonitrile, poly(styrene-co-methacrylate), poly(4-methyl-1-pentene), amorphous nylon, nylon, poly(ether sulfone), poly(ether imide), polysulfone, Dyneon^{TM} THV, etc.

In one embodiment, the light probe 176 and light guide 175 are further operable to direct the return radiation from the tooth surface to a sensor 165. The sensor 165 is operable to detect the return radiation radiated back from the tooth surface and may include, for example, at least one of a photodiode, a charge-coupled device (CCD), a photodetector, photo-multiplier tube (PMT), avalanche diodes (APD) or other photosensitive element. In one embodiment, the sensor 165 detects the return radiation and transforms it into an electrical signal corresponding to the wavelength of the return radiation. The wavelength of the return radiation then provides an indication of whether, at the investigated tooth tissue region, fluorescent materials are present or not. An example of a suitable light sensor is commercially available from TAOS, Inc., of Piano, Texas, under the designation No. TSL12S.

Since the sensor 165 is a relatively sensitive component it may be protected as well as possible from external influences and vibrations. In one embodiment, the sensor 165 is integrated into the handle section 10. In order to ensure that the return radiation is evaluated as effectively as possible, it is necessary to direct the return radiation directly into the sensor 165. Accordingly, the sensor 165 may have an opening into which the light guide 175 may be inserted and fastened to the sensor 165.

There is also integrated into the handle section 10, at least one filter 180, which filters out wavelength regions not relevant for the evaluation of the return radiation. In one embodiment, the filter 180 is configured such that the return radiation must pass through the filter 180 (i.e. filter 180 is located within the energy path between the oral cavity and the sensor 165), before reaching the sensor 165. In another embodiment, filter 180 is incorporated into sensor 165. Thus, only such light is detected and evaluated which is also for the diagnosis of fluorescent material. For example, the filter 180 may be configured such that only return radiation with suitable wavelengths is allowed to pass through the filter and on to the sensor 165, thereby excluding the light from the excitation radiation. The incorporation of filter 180 can reduce the margin for error in sensor 165.

Embodiments may include any number and variety of filters between light source 160 and sensor 165. In general, dichroic filters usually reflect portions of light which are not wanted and transmit the remainder. Bandpass filters generally filter / block wavelengths of electromagnetic energy outside of a selected interval of wavelengths. The bandpass filter may comprise a laminate structure having multiple filtering layers, e.g. a blue filter and a green filter. Longpass filters may filter / block shorter wavelengths and transmit longer wavelengths over a range of a selected spectrum, e.g. ultraviolet, visible, or infrared.

Any suitable filter known in the art may be utilized in the electric oral health detection device. Non-limiting examples include films, sheets, substrates, laminates, mirrors, mirror reflectance filters, lenses, eye glasses, eye goggles, dichroic filters, interference filters, band pass filters, optical long pass filters, filtering viewing surfaces, filtering reflective surfaces, filtered viewing devices, filtered reflective surfaces and/or combinations thereof, and other known or unknown devices operable to filter or block predetermined wavelengths of energy. A suitable example of a mirror which can be utilized in the present invention is available from American Acrylics located in Skokie, IL, and sold as Yellow Acrylic Mirror #2208. Other suitable examples of filters which can be utilized in the personal hygiene device are available from Roscolux located in Stamford, CT and sold as #312 Canary, #12 Straw, #11 Light Straw. Further examples of suitable filters for use in the present invention are available from GamColor located in Los Angeles, CA and sold as 480 Medium Yellow 88% T, and 460 Mellow Yellow 83% T. Still further suitable examples of filters for use with the present invention, although less efficient than the aforementioned filters, available from Roscolux are #06 No Color Straw, #07 Pale Yellow, #13 Straw Tint and available from GamColor 440 Very Light Straw.

The electric oral health detection device also includes a logic component 170 operable to analyze the return radiation detected by the sensor 165 and determine whether one of dental deposits mentioned above is present at the investigated tooth surface or not. The logic component 170 may include circuitry operable to, for instance, analyze fluorescence of the return radiation. The logic component 170 may also include circuitry operable to perform one or more logic or arithmetic functions for analyzing the fluorescence and intensity of the return radiation detected by the sensor 165. Thus, in operation, the logic component 170 directly evaluates the return radiation delivered thereto and determines from the detected fluorescence radiation the presence or absence of dental deposits at the irradiated tooth region. In one embodiment, if the logic component 170 determines the presence of dental deposits, a visual or audible alarm may be actuated to warn the user of the presence of a dental deposit, such as plaque.

There is also integrated into the handle section 10, an amplifier (not shown), which amplifies the return radiation detected by the sensor 165 before the return radiation is analyzed by the logic component 170. In one embodiment, the amplifier is configured such that the return radiation must pass through the amplifier before reaching the logic component 170.

The electric oral health detection device further includes an output component 195 operable to convey information from the logic component 170 to a consumer, such as a user of the toothbrush 100. The output component 195 may include at least one of a display and an audible component. In one embodiment, the output component 195 can be provided as a stand-alone display that can be mounted or placed upon on a variety surfaces, including hard surfaces such as a mirror or other glass surface, a countertop, a wall, shelf, or it may be mounted on, in, or placed within cabinetry or some other structure. In one embodiment, logic component 170 may be integrated into a stand-alone output component, such as a display.

In another embodiment, the output component 195 can be provided with a projector that can project visual information onto a surface. In another embodiment, the output component 195 can be integrated into a mirror. The output component 195 can be provided in a portable size and shape so that it can be taken with a user outside of the bathroom. Alternatively, the output component 195 can be provided as part of the electric oral health detection device as shown in Fig. 4. Examples of output components that may be used in accordance with the present disclosure are described in U.S. Patent Application Serial Nos. 61/176,618, entitled, "PERSONAL CARE SYSTEMS, PRODUCTS AND METHODS", filed on May 8, 2009; 61/180,617, entitled, "PERSONAL CARE SYSTEMS, PRODUCTS AND METHODS", filed on May 22, 2009; and U.S. Patent Application Publication No. 2008/0109973.

With reference to Fig. 2, the operating principle of the electric oral health detection device is illustrated: a) the light source 160 emits excited modulated radiation consisting of light with suitable wavelength; b) the excited radiation is guided towards a tooth surface by light guide 175 and light probe 176 to excite fluorophores in dental deposits on a user's teeth, such as the proto-porphyrin IX (PPIX) molecule; c) the teeth emit return radiation in response to the excitation radiation; d) the return radiation is guided back by light guide 175 through filter 180 towards the sensor 165; e) from the sensor 165 the return radiation passes through the amplifier 185 and into the logic component 170 for analysis.

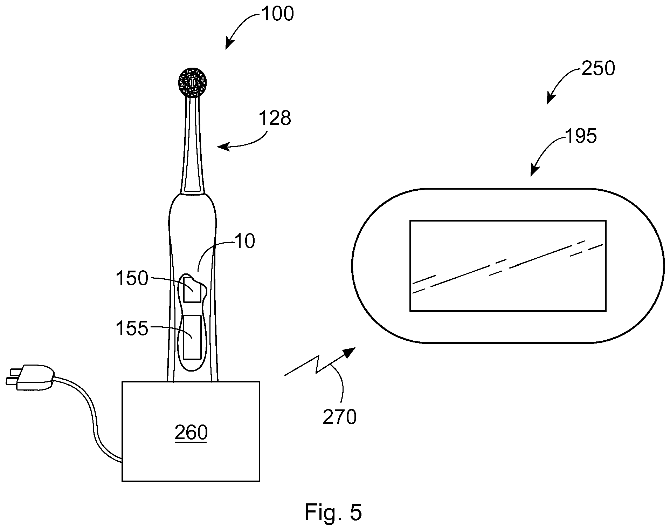

Referring to Fig. 5, an oral health detection system 250 including the electric oral health detection device in the form of electric toothbrush 100, a base 260 for receiving the electric toothbrush 100, and a visual and/or audio output component 195 that is in continuous and/or intermittent data communication with the electric toothbrush 100 and/or the base 260 before, during, and/or after use by a consumer of the electric toothbrush 100. The oral health detection system 250 can use a variety of arrangements, singly or in combination, to implement data communication between the output component 195 and the electric toothbrush 100 and/or base 260. In one embodiment, the toothbrush 100 and/or the base 260 are in wireless communication with the output component 195 via wireless data link 270. The wireless data link 270 may be based upon a suitable short range radio frequency communication technology, such as Bluetooth, WiFi (802.11 based or the like) or another type of radio frequency link, such as wireless USB at 2.4 GHz. For radio transmissions, an antenna can be mounted on a printed circuit board (PCB) disposed in the electric toothbrush 100, base 260, and/or the output component 195.

For infrared (IR) transmissions, one or more IR transmitter diodes can be mounted in the electrical toothbrush 100, the base 260, and/or the output component 195. An IR wavelength suitable for use with the present disclosure is 950 nm modulated at 36 KHz. Other wireless data communication technologies may be used.

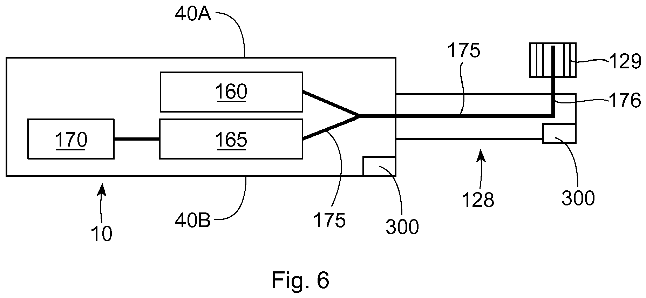

In another embodiment shown in Fig. 6, at least one indication element 300 may be disposed on the electric toothbrush 100. For example, the at least one indication element 300 may be disposed on the handle section 10; between the handle section 10 and the cleaning section 20; on the cleaning section; or a combination of these locations. In another embodiment, the at least one indication element 300 may include two or more indication elements. The at least one indication element 300 may provide a visible signal to a consumer for at least one of a plurality of conditions. For example, the visible signal, for example, blinking or flashing, may be provided when the presence of a dental deposit is determined, when the cleaning section 20 needs to be replaced, when a consumer has brushed for an adequate amount of time. Additional conditions for which a signal may be provided are also contemplated.

The at least one indication element 300 may be disposed in any suitable location on the electric toothbrush 100. For example, in some embodiments, the indication element 300 may surround the neck portion 128 or may surround the handle section 10. In another embodiment, the indication element 300 may surround a portion of the handle section 10 and/or a portion of the neck portion 128. In another embodiment, the indication element 300 may be disposed on a back-facing surface 40B of the handle section 10 and/or the neck portion 128. In another embodiment, the indication element 300 may be disposed on a front-facing surface 40A of the handle section 10 and/or the neck portion 128. In one embodiment, if the indication element 300 is disposed on the handle section 10, the indication element 300 may be integrally formed with a sealing element(s) to prevent or reduce the likelihood of water or other contaminants from entering into the handle section 10.

In one embodiment, the indication element 300 may include a light emitting diode and a translucent or transparent material to allow light to be provided to the user. Additionally, unique color combinations may be created by utilizing a colored material for the indication element 300. For example, visible light of a first color may be provided while the indication element 300 may comprise a second color. The first color and the second color may be different, for example, blue and yellow, respectively. As another example, the indication element 300 may be a first color and the visible light may comprise primarily the same color, for example, red and red.

In another embodiment, the indication element 300 may include multiple LEDs, such that a first LED may provide a first output signal for one condition, for example, presence of a dental deposit, while a second LED may provide a second output signal for a second condition, for example, adequate amount of brushing time has been reached. In another embodiment, a first LED may provide a first output signal for one condition, for example, the device is functioning normal, while a second LED may provide a second output signal for a second condition, for example presence of a dental deposit. In such embodiments, the first and second output signals may be visual and the first output may be a first color while the second output may be a second color which is different from the first color. Any suitable colors may be utilized.

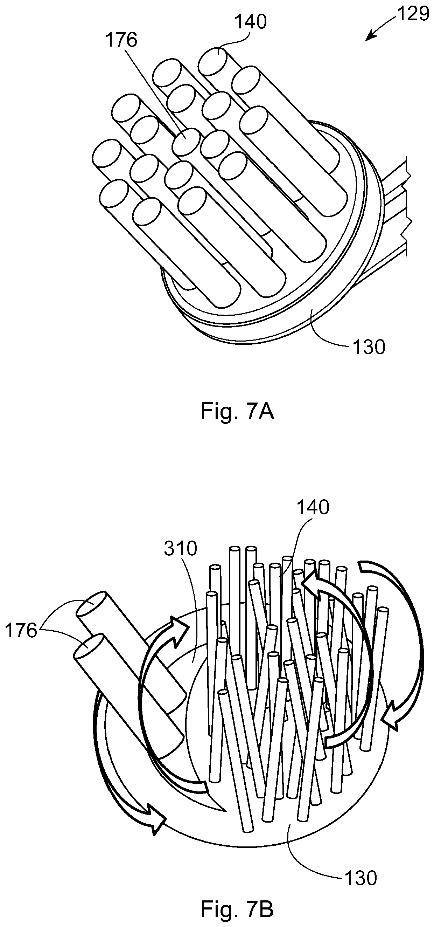

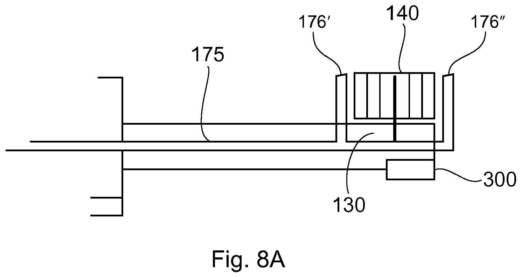

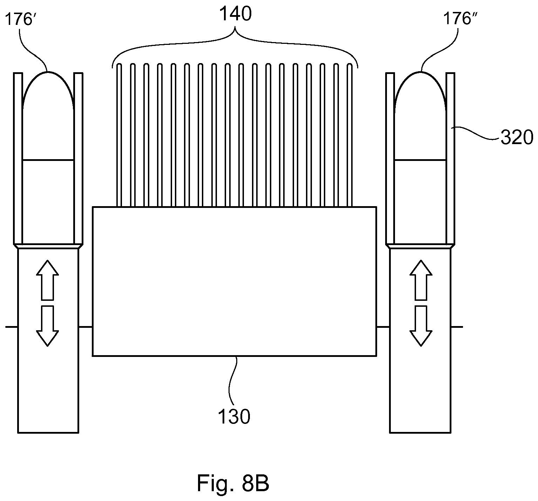

Figs. 7A-7C, 8A, and 8B illustrate various embodiments of cleaning head sections 129 and light probes 176. The cleaning head section 129 and the light probe 176 may take on a wide variety of configurations. For example, light probe 176 may be in the shape of an end rounded cylinder. It should be understood that embodiments are not limited to those configurations depicted in Figs. 7A-7C, 8A, and 8B. Accuracy of measurement is important for the electric oral health detection device of the present disclosure. In order to improve the light detection sensitivity and thus the accuracy of the measurement of the device, the light probe needs to be maintained in a relatively stationary position with respect to the dental deposit that is being examined. In one embodiment, this can be accomplished by positioning the light probe 176 at the axis of rotation of the movable carrier 130, for example at the center of the movable carrier 130. Alternatively, the light probe 176 may be fixed to the carrier 130. In either case, the light probe 176 can be considered relatively stationary in that point of time when the cleaning head section 129 passes over an individual tooth. In another embodiment, two or more light probes 176 may be located on the carrier 130.

In one embodiment, the conductivity of light probe 176 ranges from roughly 360 to 1200 nm and does not exhibit auto fluorescence within that range.

Referring to Fig. 7A, a cleaning head section 129 is depicted having a light probe 176 positioned at or near the axis of rotation of the movable carrier 130, for example at the center or near the center of the movable carrier 130, for example within a diameter of from about 0 mm to about 5 mm from the axis of rotation of the movable carrier. Light probe 176 may be stationary or non-stationary with respect to a non-movable portion of the toothbrush 100, for example, the handle portion 128. In this embodiment, the moving carrier 130 oscillates or rotates around the light probe 176. In one embodiment, light probe 176 may be positioned at the center or near the center of the movable carrier 130 and may swivel and / or rotate. In another embodiment, the light probe 176 may include a groove, a notch, or a textural difference which can provide a sensory signal to the user to indicate the location of the light probe 176 within the oral cavity.

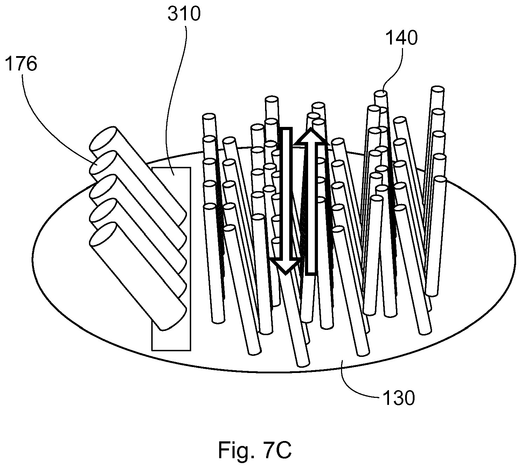

In another embodiment, as illustrated in Figs. 7B and 7C, the light probe 176 may be located at another position on the carrier 130. If the light probe 176 is not located at or near the axis of rotation, for example, the center or near the center of the movable carrier 130, the light probe should be stationary with respect to a non-movable portion of the toothbrush 100, for example, the handle portion 128. As shown in Figs. 7B and 7C, the light probe 176 includes one or more light probes and may be disposed within a recess, aperture or hole 310 that extends through the movable carrier 130. As shown in Fig. 7B, carrier 130 moves in a rotary or oscillating motion. In another embodiment, carrier 130 may move in any cleaning motion described above. As shown in Fig. 7C, the carrier 130 moves the plurality of cleaning elements 140 back and forth angularly to provide a cleaning action substantially similar to an up-down manual brushing action.

In another embodiment, as illustrated in Figs. 8A and 8B, the light probe 176 includes two light probes 176' and 176" positioned adjacent to or outside the carrier 130. As shown in Figs. 8A and 8B, light probes 176' and 176" may be positioned directly opposite each other. In one embodiment, an area adjacent to the light probe 176 may be devoid of cleaning elements 140 so that light is transmitted to the surface of the teeth without interference from the cleaning elements. In another embodiment, the light probe 176 may be a single light probe or a plurality of light probes.

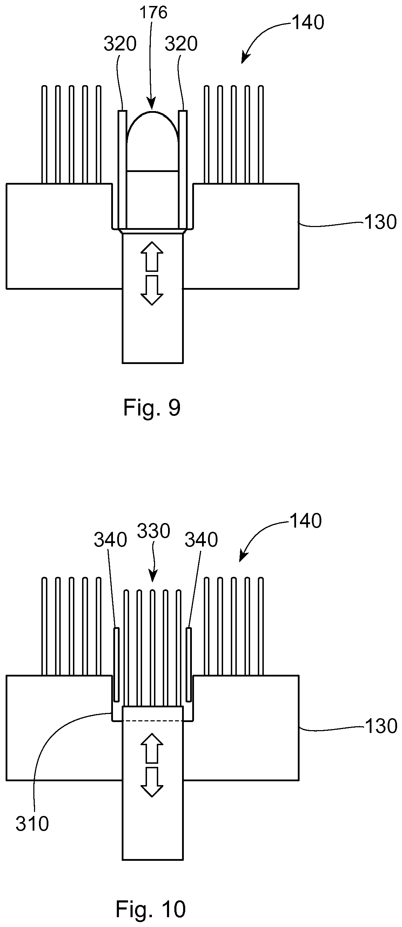

Figs. 9 and 10 illustrates embodiments of the cleaning section 129 wherein the light probe 176 is disposed within a recess, aperture or hole 310 that extends through the movable carrier 130. As shown in Fig. 9, in order to protect the light probe 176 from damage resulting from contact with the movable carrier 130 and/or a user's oral cavity, the light probe 176 may be shielded by a protective sleeve 320 or protective coating. In one embodiment, the protective sleeve 320 can comprise a variety of materials, for example, polymers, elastomers or any other soft and flexible material which can protect the light probe 176 while at the same time is also gentle on a user's oral cavity. In one embodiment, the protective sleeve 320 may completely surround the sides but not the top of the light probe 176. In another embodiment, the protective sleeve 320 may include at least two pillars that are located on opposite sides of the light probe 176. In another embodiment, the protective sleeve 320 may be a cap that extends over the light probe 176. In yet another embodiment, the protective sleeve 320 may take the form of a variety of shapes, including but not limited to, cylinders, spikes, circles, semi-circles, rectangles, squares and any combination of these shapes. In yet another embodiment, the light probe 176 may be coated with a protective coating or the light probe 176 may be constructed of a soft and flexible material in order to eliminate the need for the protective sleeve 320. In another embodiment, the light probe 176 includes two layers, an inner core for conducting light and an outer layer that surrounds the inner core, for example, a light conductive filament with a nylon sheath.

In another embodiment, the light probe 176 includes at least two layers, a core layer with higher refractive index and a cladding layer with lower refractive index which surrounds the core, for conducting light. In another embodiment, the light probe 176 includes at least three layers, a core layer with higher refractive index and a cladding layer with lower refractive index which surrounds the core, for conducting light, and an outer protective layer.

As shown in Fig. 10, the light probe 176 may include a plurality of optical fibers or filaments 330 disposed within a recess, aperture or hole 310 that extends through the movable carrier 130. In one embodiment, each of the optical fibers 330 operate as a structure for transmitting light through the interior of each fiber 330. The optical fibers 330 have substantially the same shape, flexibility, diameter as the cleaning elements 140. In one embodiment, the optical fibers 330 can be made of various polymer optical materials such as cyclic olefin copolymer, cycloolefin polymer, polycarbonate, poly(methyl methacrylate), polystyrene, allyl diglycol carbonate, poly(styrene-co-acrylonitrile, poly(styrene-co-methacrylate), poly(4-methyl-1-pentene), amorphous nylon, nylon, poly(ether sulfone), poly(ether imide), polysulfone, Dyneon™ THV, etc.

In this embodiment, the optical fibers 330 are lower than the surrounding cleaning elements 140 in order to help protect the optical fibers 330. In addition, in one embodiment, an optical filament holder 340 is connected to the movable carrier 130 and serves to secure the optical fibers on the carrier. Further, in one embodiment, the optical fibers 330 are connected to a portion of the light probe 176 located near the bottom of the recess 310 and extending within the neck portion towards the light guide 175. In another embodiment, the optical fibers 330 may be higher than the surrounding cleaning elements 140.

In the above embodiment, the movable carrier 130 may have a substantially circular shape. Alternatively, movable carrier 130 may have a generally elliptical, rectangular, oblong, oval, rounded diamond or other suitable shape.

In another embodiment, toothbrush 100 may also include a position member to measure the location and/or orientation and/or moving speed of the head of user and the location and/or orientation and/or moving speed of toothbrush 100 in order to ensure the correct technique is being used. In one embodiment, if the light source 160 receives a signal from a position member to indicate that the correct technique is being used, then the light source 160 emits excitation radiation onto a tooth surface and receives the return or response radiation from the tooth surface. If on the other hand, the position member determines that improper technique is being used, then the light source 160 will not emit excitation radiation and the toothbrush will not be able to detect dental deposits. Thus, the position member may be incorporated into the toothbrush 100 to help train the user to use proper technique when brushing. Some suitable examples of toothbrushes having position members are provided in U.S. Patent Application Serial No. 12/622/876.

In another embodiment, toothbrush 100 may also include a controller, which may include a printed circuit board with a microprocessor or an ASIC or other electrical components. The controller may have a light source inhibiting device, for example, an electronic means, which inhibits the light source 160 from turning on, i.e. emitting excitation radiation for the detection of dental deposits, when a traditional or non-diagnostic cleaning section, i.e. a cleaning section or refill that does not include optical components such as a light guide or light probe, is attached to the handle section 10. As such, with the use of a traditional cleaning section a user may continue with his or her normal brushing regimen but the ability to detect dental deposits is not possible. If the user attaches a diagnostic cleaning section 20, i.e. a cleaning section including optical components, to the handle section 10, the light source 160 is activated by means of an enabling element or coding device provided on the diagnostic cleaning section 20, for example a ring arranged at the coupling end of the cleaning section, and dental deposits may then be detected by the device during brushing. In one embodiment, a controller may have a light source activating device, for example, an electronic means, which activates the light source 160, when a diagnostic cleaning section including an enabling element is attached to the handle section 10.

Suitable examples of communication means between a brush attachment and an electric toothbrush handle when a brush attachment and handle are joined together are provided in U.S. Patent Nos. 7,024,717, 7,207,080, 7,621,015, 7,624,467, 7,661,172, 7,673,360, 7,770,251, 7,774,886, and 7,861,349. In another embodiment, an additional switch may be provided on the handle section 10 in order to activate the light source 160 when a diagnostic cleaning section is attached to the handle section 10.

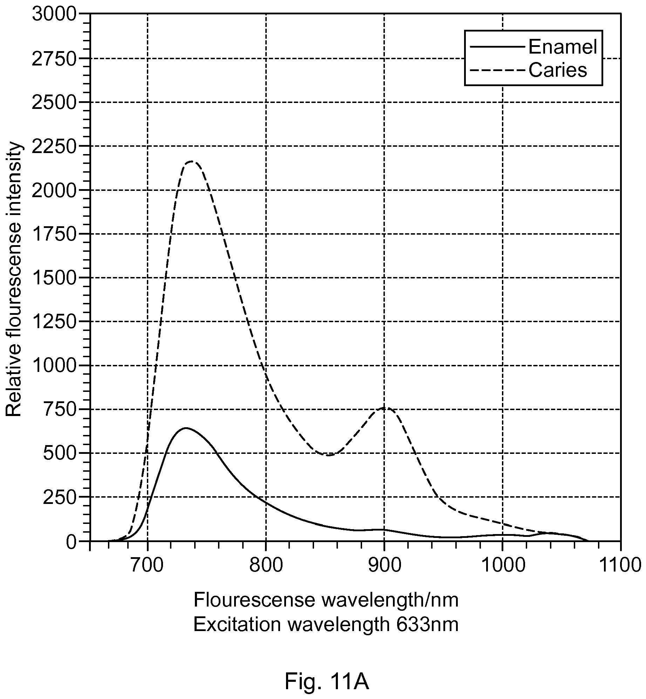

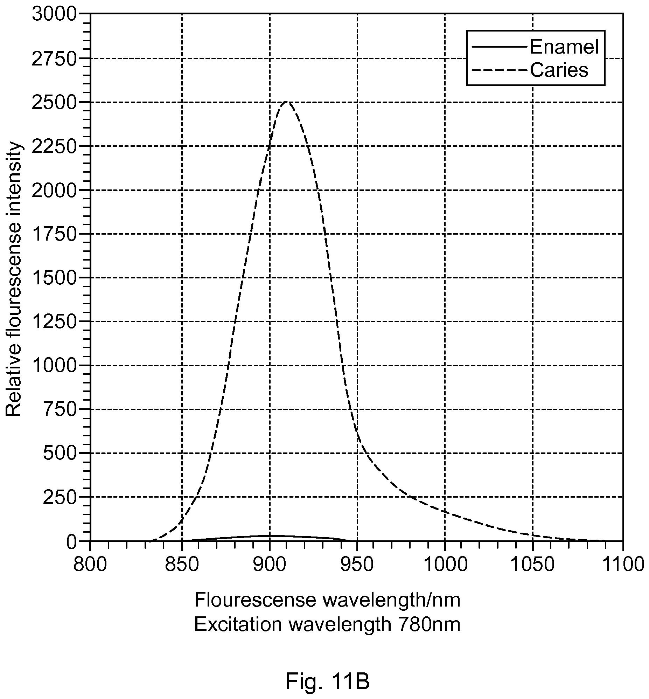

As mentioned previously, the oral health detection device evaluates fluorescence radiation arising at the tooth surface in reaction to the irradiation during normal brushing. In one embodiment, the fluorescence detection targets a bacteria metabolite often found in dental deposits, for example, the PPIX molecule. The fluorescence spectrum of a dental deposit containing PPIX molecules when excited is shown in Figs. 11A and 11B. As illustrated in Fig. 11A, the detected fluorescence intensity for dental deposit regions and healthy tooth enamel are indicated in dependence upon the fluorescence wavelength. For an excitation wavelength between about 580 nm and about 680 nm, for example, at about 633 nm, it can be seen that from the fluorescence radiation for dental deposits, two specific emission bands or peaks can be achieved, one peak at about 740 nm and one peak at about 900 nm. At these two peaks, the fluorescence intensities for dental deposits and healthy enamel are clearly distinguished. In addition, as shown in Fig. 11B, for an excitation wavelength between about 680 and about 800 nm, for example, about 780 nm, it can be seen that from the fluorescence radiation for dental deposits, one specific emission band or peak can be achieved, at about 910 nm. At this peak, the fluorescence intensities for dental deposits and healthy enamel are clearly distinguished.

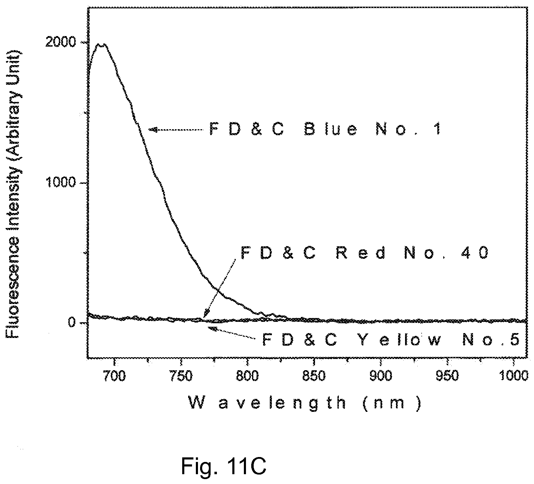

However, it has been discovered that using an excitation wavelength above about 580 nm, for example, between about 580 and about 680 nm, or any individual number within the range; and a detection range of fluorescence radiation above about 680 nm during a normal brushing regimen presents potential problems with accurately detecting dental deposits. It is common for oral care compositions such as, toothpaste, dentifrice, or tooth gel to contain dyes for purposes of improving or altering the color and/or appearance of the oral care composition. Some of these dyes however, emit a strong fluorescence emission between about 680 nm and about 1100 nm, for example, between about 680 nm and about 850 nm. For example, FD&C Blue No. 1, available from Sigma-Aldrich Co. LLC, St. Louis, Missouri, is used in a number of commercially available oral care compositions and emits a strong fluorescence emission between about 680 nm and about 850 nm at an excitation radiation of for example, about 655 nm, as shown in Fig. 11C. Fig. 11C also shows the fluorescence spectrum of FD&C Yellow No. 5 and FD&C Red No. 40. As shown in Fig. 11C, FD&C Blue No. 1 shows a strong fluorescence emission between 680 nm and 850 nm, while FD&C Yellow No. 5 and FD&C Red No. 40 exhibit negligible fluorescence emission is this wavelength range. As also shown in Fig. 11C, the fluorescence emission of all three dyes is negligible at wavelengths above 850 nm.

It is further contemplated that additional dyes used in oral care compositions also will emit a strong fluorescence emission between about 680 nm and about 850 nm.

In order to achieve optimal fluorescence detection of dental deposits, such as caries, during normal brushing routine with an oral care composition containing for example, blue dye, it is beneficial to minimize the fluorescence noise from the color dyes. A comparison of the fluorescence signals is shown below in Table 1. The measurements were made using a Diagnodent Pen Laser Caries Detector (available from KaVo Dental, Charlotte, North Carolina). The device has an excitation wavelength of about 655 nm and an emission wavelength detected of greater than 680 nm.

**Table 1**

| **Sample** | **Diagnodent-Pen Laser Measurement** |
|---|---|
| Fluorescence noise of 1:3 Diluted of Toothpaste (Crest Cavity Protection) | ~ 13 |
| Maximum fluorescence noise of healthy tooth enamel* | ∼13 |
| Maximum combined noise from enamel and diluted toothpaste (Crest Cavity Protection) | ∼26 |
| Enamel caries* | ∼ 14 to ∼ 20 |
| Deep enamel caries* | ~ 21 to -29 |
| Dentin caries* | >∼30 |

| | |
|---|---|
| *Measurement values based on Diagnodent Pen Laser user instructions | |

The laser device was used to test the fluorescence signal of diluted toothpaste that contains color dyes, for example, Crest Cavity Protection Toothpaste, available from The Procter & Gamble Company, (containing 0.0005% of FD&C Blue No. 1) diluted in water at a 1:3 ratio, to mimic the dilution by saliva during tooth-brushing. As seen in Table 1, the fluorescence noise of diluted Crest Cavity Protection Toothpaste is roughly equal to that of healthy tooth. As such, the toothpaste containing a dye contributes about 50% of the combined noise.

Table 1 shows when using a excitation radiation having a wavelength greater than about 580 nm, such as 655nm, and emission wavelength less than 850 nm, such as between 680 and 850 nm, it is possible for the maximum combined noise from enamel and diluted toothpaste to exceed the threshold of caries, which could lead to false-positive detection. Fluorescence noise from color dyes in toothpaste creates a Dye Uncertainty Factor which makes the fluorescence based diagnostics less precise and accurate.

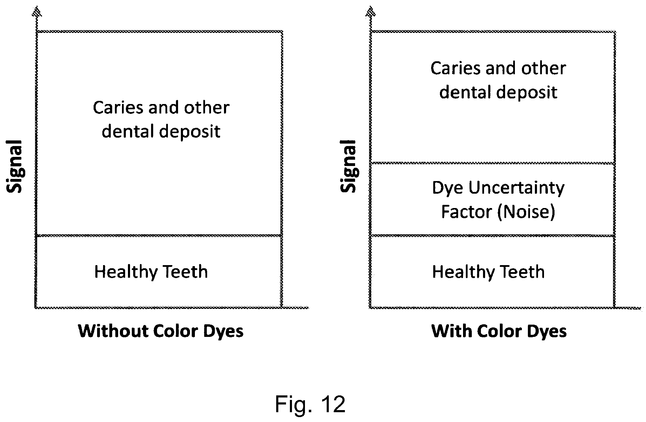

The noise or Dye Uncertainty Factor is illustrated in Fig. 12 when using an excitation wavelength between about 580 nm and about 680 nm, for example, 633nm or 655 nm, and an emission wavelengths less than 850 nm, such as between 680 and 850 nm. In order to minimize the noise or Dye Uncertainty Factor, in one embodiment, a method using an excitation wavelength between about 580 nm and about 680 nm, for example, 633nm or 655 nm, and an emission wavelengths greater than 850 nm, for example, about 900 nm may be used.

Table 2 shows the change of fluorescence signal and noise by changing the emission or detection wavelength from between about 680 nm and about 850 nm to above 850 nm.

**Table 2**

| | **at~ 740 nm** | **~ 900 nm** | **Reduction Factor** |
|---|---|---|---|
| Healthy Enamel Background noise (from Fig. 11 A) | ∼ 650 (arbitrary units) | ∼ 60 | ∼ 11 |
| Caries signal (from Fig. 11A) | ∼ 2150 | ∼ 750 | ∼ 2.9 |
| Toothpaste Dye (FD&C Blue No. 1) background noise (from Fig. 11C) | ∼ 820 | ∼ 9.5 | ∼ 86 |

Table 3 illustrates that by changing the detection wavelength from between about 680 nm and about 850 n (e.g. 740 nm), to above 850 nm (e.g. 900 nm), the fluorescence signal of healthy enamel is reduced by a factor of -11, i.e. about 10%. However, the fluorescence noise of toothpaste dye is reduced by a much greater factor of 86, i.e. about 99%. As a result, Dye Uncertainty Factor can be significantly reduced. In one embodiment, detection devices according to the present disclosure may reduce the Dye Uncertainty Factor by at least about 50%; in another embodiment, in another embodiment by at least about 70%; and in another embodiment by at least about 90%. In another embodiment, detection devices according to the present disclosure may reduce the Dye Uncertainty Factor by from about 50% to about 99%.

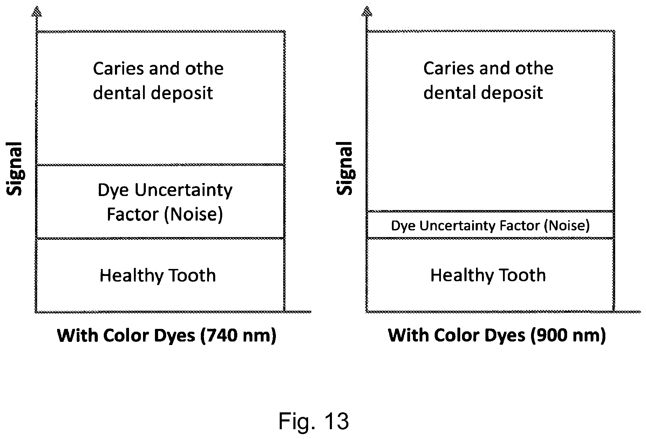

As shown in Fig. 13, the Dye Uncertainty Factor can be significantly reduced by changing the detection wavelength from between about 680 nm and about 850 nm (e.g. 740 nm), to above 850 nm (e.g. 900 nm).

As a result, if the oral care detection device is set-up to detect emission wavelengths between about 680 and about 850 nm, for example, about 740 nm, for dental deposits, such a set-up could potentially generate false positives during a brushing regimen if the user is brushing with an oral care composition containing a dye that emits fluorescence in a similar wavelength range. In one embodiment, in order to accurately detect the fluorescence of dental deposits during normal brushing, the oral health detection device can use a detection range of fluorescence radiation above about 850 nm, for example, a range of about 850 nm to about 1100 nm, or any individual number within the range. Use of a detection wavelength above about 850 nm will help to avoid any interference with a dye that is present in an oral care composition. In another embodiment, the oral health detection device can use a detection range of fluorescence radiation above about 900 nm or above about 950 nm. This enables the oral care detection device to detect the presence of dental deposits with the same precision and accuracy, i.e. the same measurement score, regardless of whether an oral care composition is present or absent during use.

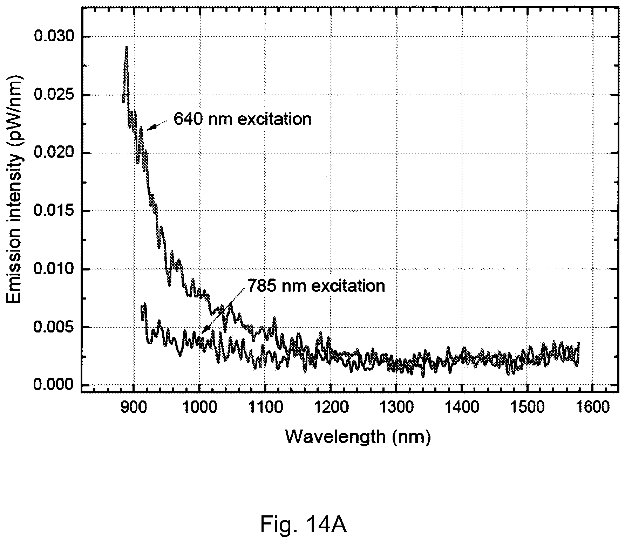

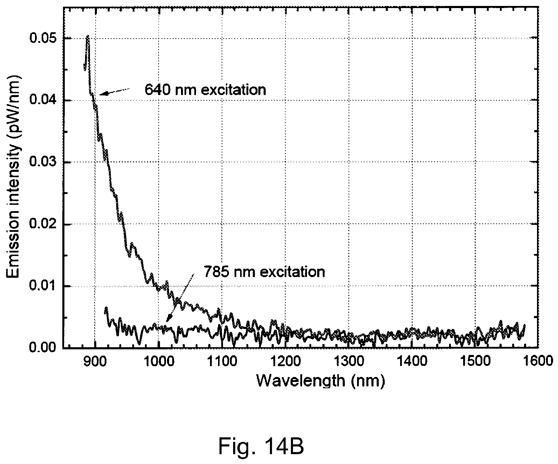

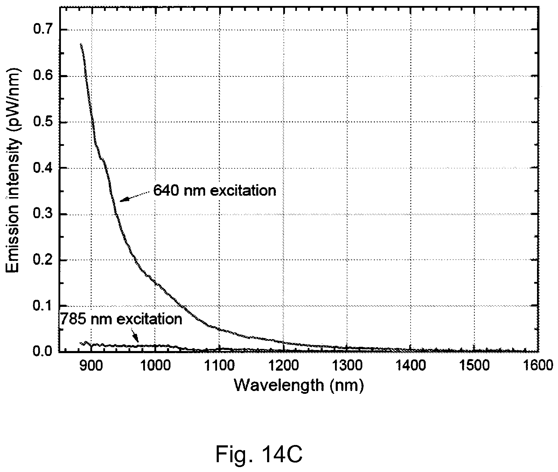

Additionally, as shown in Fig. 11B, for an excitation wavelength between about 680 and about 800 nm, for example, about 780 nm, it can be seen that from the fluorescence radiation for dental deposits, one specific emission band or peak can be achieved, at about 910 nm. At this peak, the fluorescence intensities for dental deposits and healthy enamel are clearly distinguished. Figs. 14A-C show that the emissions at 910 nm of FD&C Red No. 40 (Fig. 14A), FD&C Yellow No. 5 (Fig. 14B) and FD&C Blue No. 1 (Fig. 14C) are significantly lower using excitation wavelength between about 680 and about 800 nm, for example, about 780 nm or at about 785nm, compared with using an excitation wavelength between about 580 and about 680 nm, for example, about 640 nm or about 655 nm. Therefore, it is expected that the detection uncertainty with color dye are much lower if the device is setup to detect using an excitation wavelength between about 680 and about 800 nm, for example, about 780 nm or about 785 nm, and a detection wavelength at about 910 nm.

A method for the recognition of dental deposits is also contemplated. The method, which is not part of the invention, may be performed through use of, for instance, any of the oral health detection devices described herein. At a first step, the light source 160 contained within toothbrush 100 is activated. Next, modulated excitation radiation from the light source 160 is directed to the light guide 175 and onto a tooth of a user via the light probe 176. Next, the emission or fluorescence radiation at the tooth is received or guided back into the light probe 176 and to the light guide 175. Next, the fluorescence radiation is detected by the sensor 165. Next, the fluorescence radiation detected by the sensor 165 is analyzed by the logic component 170. Finally, the information obtained is conveyed to a user through the output component 195.

Prior to use of the oral health detection device according to the present disclosure, the device may need to be calibrated to ensure accuracy of the device. In one embodiment, calibration can be done inside the oral cavity. For example, in one embodiment, the device may take a number of measurements over a certain period of time after a user begins their normal brushing regimen. For example, the device may take five measurements during the first ten seconds of brushing. If the average of these measurements is below a certain predetermined threshold, then the average will be taken as a reference for healthy enamel. Various other combinations of the number of measurements and amount of time after brushing is initiated may also be used to calibrate the device.

The device can also apply a majority criterion by taking a plurality/majority of readings as a subset of all readings during the calibration phase with the lowest value and/or a variation below a certain value. The device can also take the lowest reading of all readings during the calibration phase which is within a range of plausible results as a reference for the healthiest piece of human enamel that was sensed during the calibration phase. Since during the start phase of a tooth cleaning procedure the light transparent elements embedded in the bristle field are expected to be covered with tooth paste, a (strictly) monotonically increasing/decreasing measured signal is expected due to the dilution of the tooth paste. In this case, the highest/lowest level of the turning point will be taken as calibration reference. If the signal approximates asymptotically a certain value, the calibration point will be taken when |*rₜ₁ - r*_{*t1*+Δ*t*}| ≤ δ (while *rₜ* are readings at a certain point in time and δ represents a threshold value) for *n* consecutive or pseudo consecutive readings.

In another embodiment, prior to brushing, a user may place the device on or over an incisor within the oral cavity and a number of measurements over a certain period of time are taken. For example, the device may take five measurements during a period of ten seconds. If the average of these measurements is below a certain predetermined threshold, then the average of these readings or a subgroup of said readings will be taken as a reference for healthy enamel. Various other combinations of the number of measurements and amount of time prior to brushing may also be used to calibrate the device.

In another embodiment, calibration may be done outside of the oral cavity. For example, a calibration area (which emits fluorescence with a certain known intensity) may be located on a docking station used for charging the device in a way that the optical channel of said device will get in optical contact with a reflection surface while the optical properties of the reflection surface have known reflection properties so that the measurement circuit of the device can be calibrated to the reflection surface. Further, the device may include a calibration button or switch located on the handle or the device executes continuous calibration cycle with or without notice of the user, while the cycle time of the calibration cycles is defined by the maximum tolerable drift of the measurement circuit. While the device is located on the docking station, the light probe on the device is orientated to face the calibration area. Calibration occurs when the calibration button or switch is activated so that light is directed from the light probe onto the calibration area and received back by the device for analysis. This measurement will then serve as a reference for healthy enamel. In another embodiment, the device may include internal calibration means that can be activated by, for example, activating a calibration button or switch located on the handle.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

1. An oral health detection device (100) for investigation of dental deposits, comprising: a cleaning section (20) having a cleaning head portion (129); a handle section (10) having a motor (150) and a drive shaft (190) having a longitudinal axis; a light source (160) for emitting excitation radiation; an assembly (175) for directing the excitation radiation to the dental region of the mouth; a light sensor (165) for receiving fluorescence radiation reflected from dental deposits; a logic component (170) operable to analyze the fluorescence radiation received by the light sensor; and a display (195) for providing to a user information concerning the presence of dental deposits; wherein the cleaning head portion includes at least one light probe (176) which is static relative to the handle section and a plurality of cleaning elements (140) arranged generally transverse to the longitudinal axis such that a cleaning motion of the plurality of cleaning elements includes a back and forth oscillating movement of the plurality of cleaning elements about the longitudinal axis and relative to the light probe;
**characterized in that** the excitation radiation has a wavelength greater than 580 nm and the light sensor is arranged for receiving fluorescence radiation having a wavelength greater than 850 nm.

2. The oral health detection device according to claim 1, wherein the plurality of cleaning elements are mounted on a movable carrier (130).

3. The oral health detection device according to claim 2, wherein the at least one light probe is disposed within a recess (310) that extends through the movable carrier.

4. The oral health detection device according to claim 3, wherein the light probe is at least partially surrounded by a protective sleeve or coating (320).

5. The oral health detection device according to anyone of the preceding claims, wherein the plurality of cleaning elements oscillate at a frequency of from 75 Hz to 300 Hz.

6. The oral health detection device according to anyone of the preceding claims, wherein the light source is coupled to a light guide (175).

7. The oral health detection device according to claim 6, wherein a portion of the light guide is located within the handle section and a portion of the light guide is located within the cleaning section.

8. The oral health detection device according to claim 6, wherein the light guide is separate from and parallel to the drive shaft.

9. The oral health detection device according to claim 6, wherein the light guide is disposed within the drive shaft.

10. The oral health detection device according to anyone of the preceding claims, further comprising a coupling section to effect coupling of the cleaning section to the handle section; and a coding device located on the cleaning section for providing a signal that enables activation of the light source contained within the handle section.

## Patentansprüche

1. Mundgesundheitserkennungsvorrichtung (100) zur Untersuchung von Zahnablagerungen, umfassend: einen Reinigungsabschnitt (20), aufweisend einen Reinigungskopfabschnitt (129); einen Griffabschnitt (10), aufweisend einen Motor (150) und eine Antriebswelle (190) mit einer Längsachse; eine Lichtquelle (160) zum Emittieren von Anregungsstrahlung; eine Anordnung (175) zum Lenken der Anregungsstrahlung auf den Zahnbereich des Mundes; einen Lichtsensor (165) zum Empfangen von Fluoreszenzstrahlung, die von Zahnablagerungen reflektiert wird; ein Logikbauteil (170), das wirksam ist, um die von dem Lichtsensor empfangene Fluoreszenzstrahlung zu analysieren; und eine Anzeige (195), um einem Benutzer Informationen bezüglich des Vorhandenseins von Zahnablagerungen zu liefern; wobei der Reinigungskopfabschnitt mindestens eine Lichtsonde (176) umfasst, die relativ zu dem Griffabschnitt statisch ist, und eine Vielzahl von Reinigungselementen (140), die im Allgemeinen quer zu der Längsachse derart angeordnet sind, dass eine Reinigungsbewegung der Vielzahl von Reinigungselementen eine oszillierende Hin- und Herbewegung der Vielzahl von Reinigungselementen um die Längsachse herum und relativ zu der Lichtsonde umfasst;
**dadurch gekennzeichnet, dass** die Anregungsstrahlung eine Wellenlänge von mehr als 580 nm aufweist und der Lichtsensor dafür ausgelegt ist, Fluoreszenzstrahlung mit einer Wellenlänge von mehr als 850 nm zu empfangen.

2. Mundgesundheitserkennungsvorrichtung nach Anspruch 1, wobei die Vielzahl von Reinigungselementen auf einem beweglichen Träger (130) befestigt sind.

3. Mundgesundheitserkennungsvorrichtung nach Anspruch 2, wobei die mindestens eine Lichtsonde innerhalb einer Vertiefung (310) angeordnet ist, die sich durch den beweglichen Träger hindurch erstreckt.

4. Mundgesundheitserkennungsvorrichtung nach Anspruch 3, wobei die Lichtsonde wenigstens teilweise von einer Schutzhülle oder -beschichtung (320) umgeben ist.

5. Mundgesundheitserkennungsvorrichtung nach einem der vorstehenden Ansprüche, wobei die Vielzahl von Reinigungselementen mit einer Frequenz von 75 Hz bis 300 Hz oszillieren.

6. Mundgesundheitserkennungsvorrichtung nach einem der vorstehenden Ansprüche, wobei die Lichtquelle an einen Lichtleiter (175) gekoppelt ist.

7. Mundgesundheitserkennungsvorrichtung nach Anspruch 6, wobei ein Abschnitt des Lichtleiters innerhalb des Griffabschnitts angeordnet ist und ein Abschnitt des Lichtleiters innerhalb des Reinigungsabschnitts angeordnet ist.

8. Mundgesundheitserkennungsvorrichtung nach Anspruch 6, wobei der Lichtleiter getrennt von und parallel zu der Antriebswelle ist.

9. Mundgesundheitserkennungsvorrichtung nach Anspruch 6, wobei der Lichtleiter innerhalb der Antriebswelle angeordnet ist.

10. Mundgesundheitserkennungsvorrichtung nach einem der vorstehenden Ansprüche, ferner umfassend einen Kopplungsabschnitt, um eine Kopplung des Reinigungsabschnitts mit dem Griffabschnitt zu bewirken; und eine Codiervorrichtung, die an dem Reinigungsabschnitt angeordnet ist, um ein Signal bereitzustellen, das die Aktivierung der innerhalb des Griffabschnitts enthaltenen Lichtquelle ermöglicht.

## Revendications

1. Dispositif de détection de santé buccale (100) pour l'examen de dépôts dentaires, comprenant : une section de nettoyage comportant une partie de tête de nettoyage (129) ; une section de manche comportant un moteur (150) et un arbre d'entraînement (190) possédant un axe longitudinal ; une source lumineuse (160) pour émettre un rayonnement d'excitation ; un ensemble (175) pour diriger le rayonnement d'excitation vers la région dentaire de la bouche ; un capteur de lumière (165) pour recevoir un rayonnement de fluorescence réfléchi par des dépôts dentaires ; un composant logique (170) exploitable pour analyser le rayonnement de fluorescence reçu par le capteur de lumière ; et un affichage (195) pour fournir à un utilisateur des informations concernant la présence de dépôts dentaires ; dans lequel la partie de tête de nettoyage inclut au moins une sonde lumineuse (176) qui est statique par rapport à la section de manche et une pluralité d'éléments de nettoyage (140) disposés de façon généralement transversale à l'axe longitudinal de telle sorte qu'un mouvement de nettoyage de la pluralité d'éléments de nettoyage inclut un mouvement oscillant vers l'arrière et vers l'avant de la pluralité d'éléments de nettoyage autour de l'axe longitudinal et par rapport à la sonde lumineuse ;
**caractérisé en ce que** le rayonnement d'excitation a une longueur d'onde supérieure à environ 580 nm et le capteur de lumière est disposé pour recevoir un rayonnement de fluorescence possédant une longueur d'onde supérieure à environ 850 nm

2. Dispositif de détection de santé buccale selon la revendication 1, dans lequel la pluralité d'éléments de nettoyage est montée sur un support mobile (130).

3. Dispositif de détection de santé buccale selon la revendication 2, dans laquelle l'au moins une sonde lumineuse est disposée au sein d'une cavité (310) qui s'étend à travers le support mobile.

4. Dispositif de détection de santé buccale selon la revendication 3, dans lequel la sonde lumineuse est au moins partiellement entourée par un manchon ou revêtement de protection (320).

5. Dispositif de détection de santé buccale selon l'une quelconque des revendications précédentes, dans lequel la pluralité d'éléments de nettoyage oscille à une fréquence allant de 75 Hz à 300 Hz.

6. Dispositif de détection de santé buccale selon l'une quelconque des revendications précédentes, dans lequel la source lumineuse est couplée à un guide lumineux (175).

7. Dispositif de détection de santé buccale selon la revendication 6, dans lequel une partie du guide lumineux est située au sein de la section de manche et une partie du guide lumineux est située au sein de la section de nettoyage.

8. Dispositif de détection de santé buccale selon la revendication 6, dans lequel le guide lumineux est indépendant de, et parallèle à, l'arbre d'entraînement.

9. Dispositif de détection de santé buccale selon la revendication 6, dans lequel le guide lumineux est disposé au sein de l'arbre d'entraînement.

10. Dispositif de détection de santé buccale selon l'une quelconque des revendications précédentes, comprenant en outre une section de couplage pour réaliser un couplage de la section de nettoyage à la section de manche ; et un dispositif de codage situé sur la section de nettoyage pour fournir un signal qui permet l'activation de la source lumineuse contenue au sein de la section de manche.
